# EUROPEAN PATENT SPECIFICATION

(11) **EP 2 286 234 B1**
(45) Date of publication and mention of the grant of the patent: **03.04.2013**
(21) Application number: 09742847.8
(22) Date of filing: 07.05.2009
(51) Int. Cl.: B82Y 30/00, G01N 21/65, G01N 33/543

(54) **NOVEL AU-AG CORE-SHELL COMPOSITE USEFUL FOR BIOSENSOR**
NEUER AU-AG-KERN-HÜLLE-VERBUNDSTOFF FÜR BIOSENSOR
NOUVEAU COMPOSITE COEUR-ENVELOPPE AU-AG CONVENANT COMME BIOCAPTEUR

(30) Priority: 07.05.2008 KR 20080042374; 06.05.2009 KR 20090039472
(43) Date of publication of application: 23.02.2011
(73) Proprietor: Seoul National University Industry Foundation, Seoul 151-919 (KR)
(72) Inventor: NAM, Jwa-Min, Seoul 151-057 (KR); LIM, Dong-Kwon, Seongnam-si Gyeonggi-do 463-706 (KR); KIM, In-Jung, Daejeon 300-770 (KR)
(74) Representative: Arends, William Gerrit
(86) International application number: PCT/KR2009/002399
(87) International publication number: WO 2009/136741

(56) References cited:
- WO-A2-2006/116742
- US-A1- 2004 086 897
- US-A1- 2005 191 665
- US-A1- 2008 096 289
- Z. LI ET AL.: "Multiple thiol-anchor capped DND-gold nanoparticle conjugates.", NUCLEIC ACIDS RESEARCH, vol. 30, no. 7, 2002, page 1558, XP055003930, Oxford UK
- M. KAHRAMAN ET AL.: "Oligonucleotide-mediated Au-Ag core-shell nanoparticles", PLASMONICS, 1 January 2009 (2009-01-01), XP55003757, Baltimore MD USA
- MATVEEVA E. ET AL.: 'Fluorescence quenching/Enhancement surface assays: Signal manipulation using silver-coated gold nanoparticles' CHEMICAL PHYSICS LETTERS vol. 454, no. IS.1-3, 10 March 2008, pages 85 - 90, XP022503697
- PANDE S ET AL.: 'Synthesis of Normal and Inverted Gold-Silver Core-Shell Architectures in beta- Cyclodextrin and their applications in SERS' J. PHYS. CHEM. C vol. 111, no. 29, 2007, pages 10806 - 10813, XP008146094
- XIE F. ET AL.: 'Homogenous silver-coated nanoparticle substrates for enhanced fluorescence detection' J PHYS CHEM B. vol. 110, no. 46, 23 November 2006, pages 23085 - 23091, XP008146105
- TANG D ET AL.: 'Ligand-functionalized core/shell Ag@Au nanoparticles label-free amperometric immune-biosensor' BIOTECHNOL & BIO-ENGINEERING vol. 94, no. 5, 2006, pages 996 - 1004, XP008146101
- GUPTA S ET AL.: 'Characterization and optimization of gold nanoparticle-based silver-enhanced immunoassays' ANAL CHEM vol. 79, no. 10, 2007, pages 3810 - 3820, XP008146120

## Description

### Technical Field

The present invention relates to an Au/Ag core-shell composite useful for biosensor; and, more particularly, to an Au/Ag core-shell composite wherein one end of a receptor is bonded with the surface of an Au nanoparticle so that a portion of the receptor is embedded into a Ag nanoparticle layer and target material recognition site of the receptor is exposed to the outside of the Ag nanoparticle layer, and a preparing method thereof.

### Background Art

Researches on methods for detecting bio materials (deoxyribonucleic acid (DNA), protein, and so on) using metal nanoparticles have been advanced since about ten years ago, and biosensors using new platform technology have been developed. Gold (Au) nanoparticle exhibits physical, chemical and optical properties due to specific Surface Plasmon Resonance (SPR). Such propertiesare mainly used in signal detection of biomolecules.

Methods using Au nanoparticle provides superior sensitivity to techniques of forming an array by attaching phosphors, and enables rapid and easy analysis and high reproduction. Furthermore, Au nanoparticle has several advantages in that it can form a stable bond with various organic molecules on their surfaces and can also maintain a stable bond state even at a high physiological salt concentration at which bio materials (oligonucleotide, protein, and so on) can maintain inherent structures. Therefore, when a biosensor using Au nanoparticle utilizes oligonucleotide (DNA fragment) or protein as a receptor, oligonucleotide can form a strong hydrogen bond with a target DNA having a complementary sequence, and protein can form a strong bond with a target protein through an antigen-antibody reaction, enabling the detection of a specific target material.

However, since Raman scattering effect of Au nanoparticle is weaker than that of Silver (Ag) nanoparticle, Au nanoparticle is low in surface enhanced Raman Scattering (SERS) effect.

On the other hand, Ag nanoparticleis superior in Raman scattering effect, but is low in stability at a high salt concentration and high temperature at which bio material can maintain its inherent structure.

Hence, many effects have been made to use characteristics of Au nanoparticles, characteristics of Ag nanoparticles, and specificity of bio materials. As a result, methods have been known which can combine Au nanoparticles, Ag nanoparticles, and DNA in various manners and detect various DNA sequences with a very low detection limit by using characteristics of Au nanoparticles, characteristics of Ag nanoparticles, and complementary hydrogen bond characteristic of DNA. In particular, methods for detecting DNA sequences through the SERS using strong optical characteristics of Ag nanoparticles are well known and widely used.

However, in order for the SERS, Ag straining is necessary after a bonding reaction of a target oligonucleotide and Au nanoparticle modified with oligonucleotide as a receptor. The SERS is possible through this process, but a nonspecific staining may occur. In this case, false positive occurs, and a background signal increases. Also, additional Ag staining is carried out.

Therefore, studies have been conducted to develop biosensors which simultaneously have advantages such as a stable bond of Au nanoparticle with bio material, and superior optical characteristics of Ag nanoparticle.

Through those studies, Ag/Au core-shell nanoparticle (see Yun Wei Cao et al., "DNA-Modified Core-Shell Ag/Au Nanoparticles", J. Am. Chem. Soc. 2001, 123, 7961-7962) and Au-Ag alloy nanoparticle were developed.

However, Au-Ag alloy nanoparticle has a low stability because an irreversible aggregation occurs at more than a high salt concentration (0.3 M NaCl) at which oligonucleotide is hybridized.

Moreover, in the case of Ag/Au core-shell composite where Ag nanoparticle forms a core and Au nanoparticle forms a shell, a more stable bond is formed because conglomerate biomaterial is attached to the Au nanoparticle shell. Thus, it is applicable to colorimetric assay. However, since Ag nanoparticle exists inside the shell, optical characteristics of Ag nanoparticle cannot be used.

Au/Ag core-shell nano material was reported in Surojit Pande et al., "Synthesis of Normal and Inverted Gold-Silver Core-Shell Architectures in β-Cyclodextrin and Their Applications in SERS", J. Phys. Chem. C 2007, 111, 10806-10813. Au/Ag core-shell can exhibit SERS effect because Ag nanoparticle forms a shell. It has been reported that Ag/Au core-shell nano material cannot almost detect signals in Raman, but Au/Ag core-shell nano material can detect signals more sensitive in Raman. However, in order for application to biosensors using the useful optical characteristics of Au/Ag core-shell nano material, it is necessary to stably bond bio material, such as oligonucleotide or protein, as a receptor on a surface of Ag nanoparticle forming a shell. However, such a method is not disclosed in Surojit Pande et al..

Researches have been conducted to improve stability by strongly combining bio material as a receptor on the surface of Ag nanoparticle. It was reported that, when oligonucleotide is used as bio material being a receptor, oligonucleotide sequence to which dithiol or tetrathiol instead of monothiol is introduced as a functional group is combined on the surface of pure Ag nanoparticle, thereby improving the stability of Ag nanoparticle forming the above bond (Zhi Li et al., "Multiple thiol-anchor capped DNA-gold nanoparticle conjugates", Nucleic Acids Research, 2002, Vol. 30, No. 7, 1558-1562). In this case, however, since oligonucleotide bonded with typical monothiol that can be easily synthesized is not used, a complicated oligonucleotide synthesis process is additionally accompanied. Thus, in spite of superior optical characteristics of Ag nanoparticle, the above-mentioned technology is not widely used in nano bio sensing fields.
M. Kahraman et al., Plasmonics, 1 January 2009 (2009-01-01), discloses oligonucleotide-mediated Au-Ag core-shell nanoparticles.
US2005/191665 discloses composite organic-inorganic nanoclusters which include metal particles and a Raman-active organic compound.
Matveeva E et al., Chemical Physics Letters, vol. 454, no. IS.1-3, 10 March 2008 (2008-0310), pages 85-90, discloses silver-coated gold nanoparticles in fluorescence quenching-enhancement surface assays.
US2008/096289 discloses a nanoparticle having a self assembly monolayer of molecules as a shell on the nanoparticle. The monolayer may include organic molecules.
W02006/116742 discloses conjugate compositions that include a specific-binding moiety covalently coupled to a nanoparticle.
US2004/086897 discloses reagents comprising particles with at least one Raman dye and a specific binding member bound thereto.
Xie F et al. J Phys Chem B., vol. 110, no. 46, 23 November 2006 (2006-11-23), pages 23085-23091, discloses homogeneous silver-coated nanoparticle substrates for enhanced fluorescence detection.
Tang D et al Biotechnol & Bio-Engineering, vol. 94, no. 5, 2006, pages 996-1004, reports on a low-temperature method for generating particles consisting of a core of Ag and a monolayer shell of Au that can be readily functionalised with immunoprotein molecules.
Gupta S et al. Anal Chem. Vol. 79, no. 10, 2007, pages 3810-3820, discloses gold nanoparticle-based silver-enhanced immunoassays.

Therefore, there is a need for biosensors that can use advantages of both of the Ag nanoparticle and the Au nanoparticle and maintain stability in bonding of bio material as a receptor.

### DISCLOSURE

### TECHNICAL PROBLEM

An embodiment of the present invention is directed to providing an Au/Ag core-shell composite, a method for preparing the same, and a biosensor using the same.

Other objects and advantages of the present invention can be understood by the following description, and become apparent with reference to the embodiments of the present invention. Also, it is obvious to those skilled in the art of the present invention that the objects and advantages of the present invention can be realized by the means as claimed and combinations thereof.

### TECHNICAL SOLUTION

In accordance with an aspect of the present invention, there is provided an An Au/Ag core-shell composite including an Au nanoparticle; an Ag nanoparticle layer surrounding the Au nanoparticle; and a receptor having a target material recognition site bondable or reactable with a target material, wherein one end of the receptor is bonded on the surface of the Au nanoparticle, so that a portion of the receptor is embedded into the Ag nanoparticle layer, and the target material recognition site is exposed to the outside of the Ag nanoparticle layer.

In accordance with another aspect of the present invention, there is provided a method for preparing an Au/Ag core-shell composite, the method including: bonding one end of a receptor, which has a target material recognition site bondable or reactable with a target material, on the surface of an Au nanoparticle; forming an Ag nanoparticle layer on the surface of the Au nanoparticle so that a portion of the receptor is embedded into the Ag nanoparticle layer, and the target material recognition site of the receptor is exposed to the outside of the Ag nanoparticle layer.

In accordance with still another aspect of the present invention, there is provided a biosensor for detecting a target material to be bonded or reacted with a target material recognition site of a receptor comprising the Au/Ag core-shell composite.

In accordance with further aspect of the present disclosure there is provided a method for detecting a target material to be bonded or reacted with a target material recognition site of a receptor by using the biosensor.

### Advantageous Effects

In accordance with the embodiments of the present invention, Au nanoparticle and organic molecule in the Au/Ag core-shell composite can be stably bonded together, and the Au/Ag core-shell composite can exhibit superior optical characteristics of Ag nanoparticle. Thus, the Au/Ag core-shell composite exhibits stable performance under conditions of high salt concentration, high temperature, and long-term storage. Since thebiosensor using the Au/Ag core-shell composite effectively performs the detection of target bio material, the Au/Ag core-shell composite will be variously used in medical and pharmacy fields where the detection of bio material is important.

### Brief Description of Drawings

Fig. 1 is a schematic view showing an Au/Ag core-shell composite in accordance with Example 3 of the present invention.

Fig. 2 is a schematic view showing an Au/Ag core-shell composite in accordance with Example 6 of the present invention.

Fig. 3 is a schematic view showing a method for preparing the Au/Ag core-shell composite in accordance with Example 3 of the present invention.

Fig. 4 is a schematic view showing a method for preparing the Au/Ag core-shell composite in accordance with Example 6 of the present invention.

Fig. 5 shows UV spectrum of the Au/Ag core-shell composite in accordance with Example 3 of the present invention.

Fig. 6 is a transmission electron microscope (TEM) image of the Au/Ag core-shell composite in accordance with Example 3 of the present invention.

Fig. 7 is an enlarged image of Fig. 6.

Fig. 8 shows the EDX analysis result in accordance with Example 3 of the present invention.

Fig. 9 shows UV spectrum of an Au/Ag core-shell composite in accordance with Example 4 of the present invention.

Fig. 10 is a TEM image of the Au/Ag core-shell composite in accordance with Example 4 of the present invention.

Fig. 11 shows UV spectrum of the Au/Ag core-shell composite in accordance with Example 6 of the present invention.

Fig. 12 is a TEM image of the Au/Ag core-shell composite in accordance with Example 6 of the present invention.

Fig. 13 shows the variation of extinction of an Au/Ag core-shell composite in accordance with Example 7 of the present invention, according to amounts of AgNO₃ and hydroquinone.

Fig. 14 shows UV spectrum of a simple mixture of Au nanoparticle and Ag nanoparticle and an Au-Ag core-shell composite in Example 7.

Fig. 15 shows the result of the stability test in Example 8.

Fig. 16 shows a base sequence of oligonucleotides A and B contained in the Au/Ag core-shell composite used in Example 9, and a target oligonucleotide having a complementary base sequence.

Fig. 17 shows the colorimetric assay result in Example 9.

Fig. 18 shows thevariation of melting point with respect to time in Example 9.

Figs. 19 and 20 are TEM images of Example 10.

### Best Mode for Carrying out the Invention

The advantages, features and aspects of the invention will become apparent from the following description of the embodiments with reference to the accompanying drawings, which is set forth hereinafter.

An Au/Ag core-shell composite in accordance with an embodiment of the present invention includes: an Au nanoparticle; an Ag nanoparticle layer surrounding the Au nanoparticle; and a receptor having a target material recognition site bondable and reactable with a target material. One end of the receptor is bonded with the surface of the Au nanoparticle, and a portion of the receptor is embedded into the Ag nanoparticle layer. The target material recognition site of the receptor is exposed to the outside of the Ag nanoparticle layer.

The Au nanoparticle can form a stable bond with organic molecules because of its strong affinity with the organic molecules, and has a high stability even at a high physiological salt concentration at which biomacromolecules such as DNA or proteins can maintain their inherent structures. Therefore, by forming the core of the core-shell composite with the Au nanoparticle and bonding the receptor on the surface of the Au nanoparticle, stable physical characteristics are exhibited even at a high salt concentration and high temperature. Thus, the Au/Ag core-shell composite in accordance with an embodiment of the present invention can be applied to biosensors under various environments.

In accordance with an embodiment of the present invention, the size of the Au nanoparticle may be in a range of about 1 nm to about 1,000 nm, specifically in a range of about 1 nm to about 500 nm, but is not limited thereto.

Furthermore, Au nanoparticle forming a core of the Au/Ag core-shell composite in accordance with an embodiment of the present invention may be one nanoparticle or combination of two or more nanoparticles.

In accordance with an embodiment of the present invention, Au nanoparticles that are combination of two or more nanoparticles may be formed using a complementary hydrogen bond between oligonucleotides. For example, if two Au nanoparticles bonded with an oligonucleotide (A) and an oligonucleotide (B) respectively, capable of complementary bond with a specific oligonucleotide (T), are bonded with the specific oligonucleotide (T), the two Au nanoparticles can form a dimer.

A method for forming a combination of two or more nanoparticles, such as a dimer, a trimer, and so on, is not limited to the use of complementary bond between the oligonucleotides, but may be properly selected by those skilled in the art, considering experimental conditions.

Even when the Au nanoparticle has a combination of two or more particles, an Ag nanoparticle layer may be formed on the respective particles, as described later.

In accordance with an embodiment of the present invention, the Ag nanoparticle layer may be formed to surround the Au nanoparticle. By forming the Ag nanoparticle layer as a shell of the core-shell composite, the composite can have superior optical characteristics. That is, the Ag nanoparticle layer has contact points called a hot-spot or a nano-junction between Ag nanoparticles, and SERS phenomenon appears further strongly at such positions. Due to those, high selectivity and sensitivity are provided, and multiple detection of a target material is possible using various Raman tags.

The Ag nanoparticle layer may be so thick as to cover a part of the receptor and expose the target material recognition site of the receptor to the outside. Specifically, the thickness of the Ag nanoparticle layer may be changed according to kinds of the spacer and receptor.

In accordance with an embodiment of the present invention, the receptor may include the target material recognition site bondable or reactable with the target material.

In accordance with an embodiment of the present invention, the bond or reaction of the receptor and the target material may be formed by, but is not limited to, a covalent bond, a hydrogen bond, an antigen-antibody reaction, or an electrostatic attraction.

Nonrestricted examples of the receptor may be one or more selected from the group consisting of enzyme substrate, ligand, amino acid, peptide, protein, antibody, nucleic acid, oligonucleotide, lipid, cofactor, and carbohydrate.

In accordance with an embodiment of the present invention, one end of the receptor is bonded on the surface of the Au nanoparticle, and a portion of the receptor is embedded into the Ag nanoparticle. The target material recognition site is exposed to the outside of the Ag nanoparticle layer.

In accordance with an embodiment of the present invention, the receptor may include a spacer site where one end thereof is bonded on the surface of the Au nanoparticle, and another end thereof is bonded with the target material recognition site.

When the receptor further includes the spacer site, the spacer site where one end is bonded on the surface of the Au nanoparticle may be embedded into the Ag nanoparticle layer, the target material recognition site bonded with another end of the spacer site may be exposed to the outside of the Ag nanoparticle layer.

The spacer site of the receptor may serve to ensure a space in order that the target material recognition site of the receptor bonded or reacted with the target material is not covered by the Ag nanoparticle layer.

Nonrestrictive examples of the spacer site of the receptor include: a base sequence consisting of one base selected from adenine, guanine, cytosine, and thymine; polyethylene glycol (PEG); or a combination of the base sequence and the polyethylene glycol.

The number of bases of the base sequence consisting of one base selected from adenine, guanine, cytosine, and thymine, or length of the polyethylene glycol is not limited, and may be properly selected in order that the Ag nanoparticle layer may be formed on the Au nanoparticle, and the target material recognition site may be exposed to the outside of the Ag nanoparticle layer.

In accordance with an embodiment of the present invention, the Au/Ag core-shell composite may include DNA as a receptor, and the spacer site of the receptor may include: a base sequence consisting of one base selected from adenine, guanine, cytosine, and thymine; polyethylene glycol (PEG); or a combination of the base sequenceand the polyethylene glycol.

The Au/Ag core-shell composite may further include a spacer molecule mediating the bonding of the receptor and the Au nanoparticle.

The spacer molecule of which one end is bonded with the surface of the Au nanoparticle is embedded into the Ag nanoparticle layer, and the target material recognition site bonded with another end of the spacer molecule is exposed to the outside of the Ag nanoparticle layer.

Like the above-mentioned spacer site of the receptor, the spacer molecule may serve to ensure a space in order that the target material recognition site of the receptor bonded or reacted with the target material is not covered by the Ag nanoparticle layer.

Nonrestrictive examples of the spacer molecule include at least one selected from the group consisting of protein A, protein G, and protein A/G.

In accordance with an embodiment of the present invention, the receptor may be antibody or protein and the spacer molecule may be at least one selected from the group consisting of protein A, protein G, and protein A/G.

In the above-mentioned embodiment of the present invention, the Au/Ag core-shell composite basically has the Au/Ag core-shell structure, and a portion of the receptor having one end bonded on the surface of the Au nanoparticle is buried in the Ag nanoparticle layer, and the target material recognition site of the receptor is exposed to the outside of the Ag nanoparticle layer.

In accordance with an embodiment of the present invention, because of stable bond between the Au nanoparticle and the receptor being biomacromolecule, the Au/Ag core-shell composite can exhibit stable physical properties at a high salt concentrationand temperature that are required for use as biosensors, and can alsoefficiently use the signal amplification characteristic by using optical properties of the Ag nanoparticle layer. Therefore, the Au/Ag core-shell composite can be applied to detect various bio materials with ultra-high sensitivity and can obtain a further quantitative detection result.

In accordance with an embodiment of the present invention, the bonding between the surface of the Au nanoparticle and the receptor, the spacer site of the receptor or the spacer molecule may be formed by a covalent bond, an electrostatic attraction or the like.

Also, in accordance with an embodiment of the present invention, the receptor, the spacer site of the receptor or the spacer molecule may further include a functional group that mediates the bonding with the Au nanoparticle.

Examples of the functional group may be one or more selected from the group consisting of amine group, carboxyl group, thiol group, and phosphate group.

Meanwhile, a method for preparing an Au/Ag core-shell composite in accordance with an embodiment of the present invention includes: bonding one end of a receptor, which has a targetmaterial recognition site bondable or reactable with a target material, on the surface of an Au nanoparticle; and forming an Ag nanoparticle layer on the surface of the Au nanoparticle so that a portion of the receptor is embedded into the Ag nanoparticle layer and the target material recognition site of the receptor is exposed to the outside of the Ag nanoparticle layer.

In accordance with an embodiment of the present invention, the Au nanoparticle may be used with or without a surface stabilizer added. Also, the Au nanoparticle may be used in a state of being dispersed in an organic solvent or an aqueous solution. Preferably, the Au nanoparticle is used in a state of being dispersed in an aqueous solution.

In accordance with an embodiment of the present invention, the Au nanoparticle can form a stable bond with organic molecules because of strong affinity with the organic molecules. For example, one end of the receptor having a target material recognition site bondable or reactable with a target material may be bonded on the surface of the Au nanoparticle by a covalent bond or an electrostatic attraction.

In accordance with an embodiment of the present invention, forming the Ag nanoparticle layer on the surface of the Au nanoparticle bonded with the receptor may be performed by an Ag ion reduction reaction. That is, an Ag ion (Ag⁺) source and a reducing agent are added to a solution of Au nanoparticle bonded with the receptor and reacted to form the Ag nanoparticle layer on the surface of the Au nanoparticle.

In the reduction reaction, the concentration of the solution of Au nanoparticle bonded with the receptor may be in a range of about 0.1 nM to about 100 nM, specifically 1.0 nM to 10 nM.

The Ag ion (Ag⁺) source usable in the reduction reaction may be an Ag salt, specifically a water-soluble Ag salt, more specifically AgNO₃.

Also, the reducing agent may be hydroquinone, ascorbate, citrate, or metal borohydride such as sodium borohydride. Preferably, the reducing agent is hydroquinone.

A reaction solvent useable in the Ag ion reduction reaction may be an organic solvent, an aqueous solvent, or a mixture thereof. Preferably, the reaction solvent is an aqueous solvent.

Also, a reaction temperature in the Ag ion reduction reaction may be in a range of about -20°C to about 100°C. Preferably, the reaction temperature is in a range of about 15°C to about 35°C. If the reaction temperature is below -20°C, Ag nanoparticles may be aggregated. If the reaction temperature exceeds 100°C, the receptor such as DNA or protein may be damaged.

In the reduction reaction, the thickness of the Ag nanoparticle layer may be controlled by adjusting amounts of the Ag ion (Ag⁺) source and thereducing agent. Therefore, as mentioned above, a portion of the receptor is embedded into the Ag-nanoparticle layer, and the target material recognition site of the receptor is exposed to the outside of the Ag nanoparticle layer. In this way, the Ag nanoparticle layer is formed on the surface of the Au nanoparticle to a proper thickness.

The concentrations of the Ag ion (Ag⁺) source and the reducing agent may be adequately selected according to specific experimental conditions (thickness of the Ag nanoparticle layer, and so on), for example, they may be in a range of about 0.00001 M to about 10 M, but are not limited thereto. If exceeding the above range, the thickness of the Ag nanoparticle layer may increase nonspecifically. If less than the above range, the Ag nanoparticle layer may not be properly formed.

In accordance with an embodiment of the present invention, the formation of the Ag nanoparticle layer may be preferably performed by a mild reaction, for example, a mild vortexing with light being blocked, in order not to affect the stability of the receptor bonded with the Au nanoparticle.

In accordance with another embodiment of the present invention, the method for preparing the Au/Ag core-shell composite may further include connecting a spacer site to the target material recognition site of the receptor. According to this method, one end of thespacer site of the receptor is bonded on the surface of the Au nanoparticle. By forming the Ag nanoparticle layer on the surface of the Au nanoparticle, the spacer site of the receptor is embedded into the Ag nanoparticle layer and the target material recognition site of the receptor is exposed to the outside of the Ag nanoparticle layer. In this way, the Au/Ag core-shell is prepared.

In accordance with still another embodiment of the present invention, the method for preparing the Au/Ag core-shell composite may further include attaching a spacer molecule to the surface of the Au nanoparticle. The spacer molecule mediates the bond between the surface of the Au nanoparticle and the receptor. According to this method, the spacer molecule is attached to the surface of the Au nanoparticle, and the receptoris bonded with the spacer molecule. By forming the Ag nanoparticle layer on the surface of the Au nanoparticle, the spacer molecule is embedded into the Ag nanoparticle layer and the target material recognition site of the receptor is exposed to the outside of the Ag nanoparticle layer. In this way, the Au/Ag core-shell is prepared.

In accordance with an embodiment of the present invention, the bond of the receptor, the spacer site of the receptor or the spacer molecule on the surface of the Au nanoparticle may be achieved by mediation of the functional group.

Examples of the functional group may be one or more selected from the group consisting of amine group, carboxyl group, thiol group, and phosphate group.

Meanwhile, an embodiment of the present invention relates to a biosensor for detecting a target material to be bonded or reacted with a target material recognition site of a receptor comprising the above-mentioned Au/Ag core-shell composite in accordance with an embodiment of the present invention.

Due to the use of the Au/Ag core-shell composite, the biosensor in accordance with an embodiment of the present invention has the stability at a high salt concentration and temperature, the superior optical characteristics, and the specific bonding characteristic with respect to the target material. Thus, the biosensor can perform multiple detections with respect to the target materials such as various bio materials with high efficiency and sensitivity.

Meanwhile, an embodiment of the present disclosure relates to a method for detecting a target material to be bonded or reacted with a target material recognition site of a receptor by using the above-mentioned biosensor in accordance with an embodiment of the present invention.

The detection may be performed by one or more selected from the group consisting of a colorimetric assay method, an UV spectroscopic method, a Raman spectroscopic method, an optical microscopy method, an electric sensing method, and a scanometric method.

In accordance with an embodiment of the present invention, the target material is a material bondable or reactable with the target material recognition site of the receptor of the biosensor. Preferably, the target material is a bio material. More preferably, the target material is enzyme, protein, nucleic acid, oligonucleotide, oligosaccharide, peptide, amino acid, carbohydrate, lipid, cell, cancer cell, cancer stem cell, antigen, aptamer, or other bio-derived materials.

In accordance with an embodiment of the present invention, when DNA or oligonucleotide is used as the receptor, the bond between the target material and the target material recognition site of the receptor may be formed by a complementary hydrogen bond. When protein is used as the receptor, the bond between the target material and the target material recognition site of the receptor may be formed by an antigen-antibody reaction.

The biosensor using the Au/Ag core-shell composite in accordance with the embodiment of the present invention can detect the desired specific target material selectively and specifically.

**Example**

Hereinafter, specific examples of the present invention will be described in detail with reference to the accompanying drawings. The following examples are merely exemplary, and the present invention is not limited to them.

Au nanoparticle used herein was purchased from Ted pella (Redding, CA, USA), and an AgNO₃ solution as Ag ion (Ag⁺) source and a hydroquinone solution as a reducing agent was purchased from BBI international (Cardiff, UK). Oligonucleotide bonded with thiol group was purchased from IDT (Coralville, IA, USA) and thiol group was deprotected. Also, protein A and antibody was purchased from piercenet.com (USA). H₂O used in the experiment was nanopure water.

**Example 1: Preparation of Oligonucleotide**

3'-alkylthiol modified oligonucleotide, 3'-HO-(CH₂)₃-S-S-(CH₂)₃-A₁₀-PEG₁₈-CTCCCTAATAACAAT-5', which was purchased from IDT, was added to 0.1 M dithiothretol and a deprotection reaction was performed by leaving it at room temperature for 2 hours.

Oligonucleotide A (3'-HS-(CH₂)₃-A₁₀-PEG₁₈-CTCCCTAATAACAAT-5') was prepared by purifying the deprotected solution while passing it through NAP-5 column (Sephadex G-25 medium, DNA grade).

AgNO₃(50 mM) dissolved in distilled water was added to 5'-alkylthiol modified oligonucleotide (5'-HO-(CH₂)₃-S-S-(CH₂)₆-PEG₁₈-ATCCTTATCAATATT-3') and left for 20 minutes, and the generated precipitation was removed by adding dithiothretol (10 mg/mℓ) for 5 minutes.

Oligonucleotide B (5'-HS-(CH₂)₆-A₁₀-PEG₁₈-ATCCTTATCAATATT-3') was prepared by purifying supernatant while passing it through NAP-5 column (Sephadex G-25 medium, DNA grade).

By measuring extinction using a UV-visible spectrometer, an amount of oligonucleotide inside the solution was quantified.

**Example 2: Bonding of Oligonucleotide (receptor) on Surface of Au Nanoparticle**

The oligonucleotide deprotected through the procedure of Example 1 and bonded with thiol group and spacer site was added to 1 mℓ of the 3.8 nM solution of Au nanoparticle with a diameter of 15 nm and was mixed by shaking at room temperature for more than 12 hours.

The composition of the solution was adjusted so that the concentration of phosphate becomes 9 mM and the concentration of sodium dodecyl sulfonate becomes about 0.1 %. After additional agitation for 30 minutes, the final salt concentration was adjusted to be 0.3 M NaCl.

After leaving for more than 12 hours, the solution is centrifuged and the supernatant was discharged. Then, 1 mℓ of 0.3 M phosphate solution (10 mM PB, 0.3 M NaCl) was added and diluted. Those steps were repeated two times.

In this way, oligonucleotide was bonded on the surface of the Au nanoparticle.

**Example 3: Preparation (1) of Au/Ag Core-shell Composite bonded with Oligonucleotide**

The concentration of the solution of the Au nanoparticle bonded with oligonucleotide, which was synthesized in Example 2, was calculated using the extinction measured by the UV-visible spectrometer. The concentration of the solution was adjustedto 1 nM by concentrating or diluting the solution according to the result.

To 250 *µ*ℓ of the solution, AgNO₃ solution (12 *µ*ℓ*)* diluted 10 times with distilled water, and hydroquinone solution (12 *µ*ℓ) diluted 10 times with distilled water were sequentially added and then agitated for 30 minutes.

Thereafter, the extinction of the solution was measured by the UV-visible spectrometer. After leaving the solution at room temperature till there is no change in the extinction, it was centrifuged to remove the supernatant and was diluted with distilled water. Then, the solution was again centrifuged to remove the supernatant and was diluted with 250 *µ*ℓ of distilled water.

In this way, the Au/Ag core-shell composite bonded with oligonucleotide in accordance with the embodiment of the present invention was prepared.

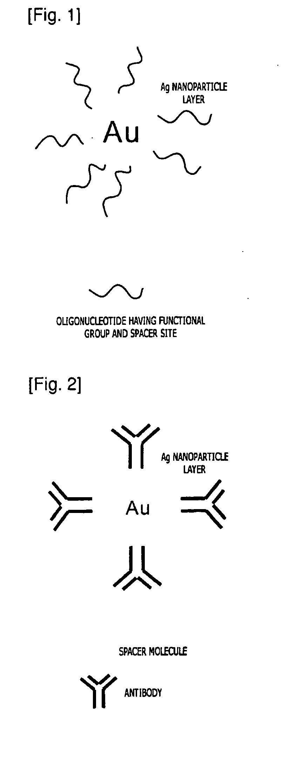

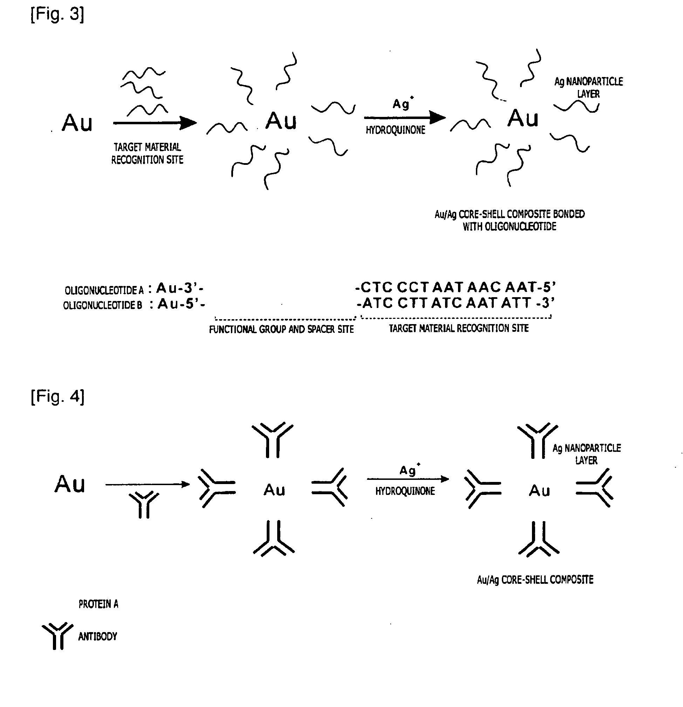

Fig. 1 is a schematic view showing the Au/Ag core-shell composite, and Fig. 3 is a schematic view showing the method for preparing the Au/Ag core-shell composite.

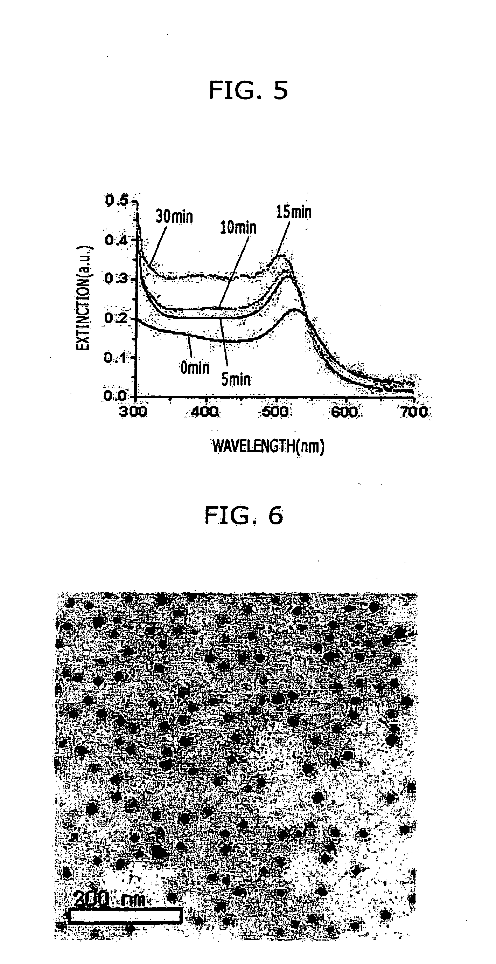

Fig. 5 shows variation in extinction of the solution measured by the UV-visible spectrometer with respect to time. As can be seen from Fig. 5, the extinction was not substantially varied after reaction for about 30 minutes.

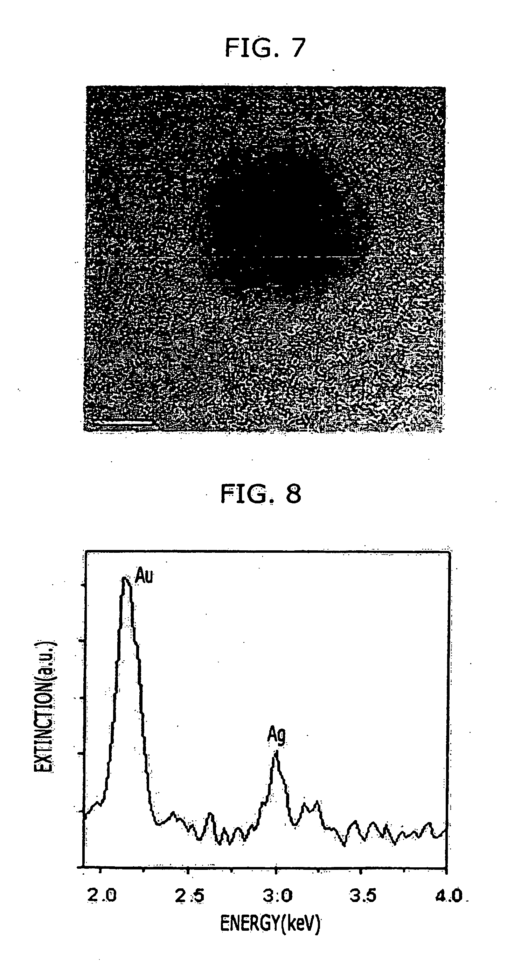

Furthermore, theshape and size of the prepared Au/Ag core-shell composite were confirmed using a transmission electron microscope (TEM) (see Figs. 6 and 7). The Au/Ag core-shell composite of Example 3 was spherical in shape and was about 16 nm to about 17 nm in size, and the Ag nanoparticle layer was about 1.5 nm in thickness.

Moreover, by analyzing the solution using an energy dispersive X-ray microanalysis (EDX), the composition ratio of Au nanoparticle to Ag nanoparticle in the prepared Au/Ag core-shell composite was confirmed.

According to the EDX analysis of Fig. 8, silver (Ag) atoms and gold (Au) atoms in the Au/Ag core-shell composite were 25 % and 75 %, respectively. This result was identical to the TEM analysis result of Figs. 6 and 7.

**Example 4: Preparation (2) of Au/Ag Core-shell Composite bonded with Oligonucleotide**

The concentration of the solution of the Au nanoparticle bonded with oligonucleotide, which was synthesized in Example 2, was calculated using the extinction measured by the UV-visible spectrometer. The concentration of the solution was adjusted to 1 nM by concentrating or diluting the solution according to the result.

To the 250 *µ*ℓ solution, AgNO₃ solution (24 *µ*ℓ) diluted 10 times with distilled water, and hydroquinone solution (24 *µ*ℓ) diluted 10 times with distilled water were sequentially added and then agitated for 30 minutes.

Thereafter, the extinction of the solution was measured by the UV-visible spectrometer. After leaving the solution at room temperature till there is no change in the extinction, it was centrifuged to remove the supernatant and was diluted with distilled water. Then, the solution was again centrifuged to remove the supernatant and was diluted with 250 *µ*ℓ of distilled water.

In this way, the Au/Ag core-shell composite bonded with oligonucleotide in accordance with the embodiment of the present invention was prepared.

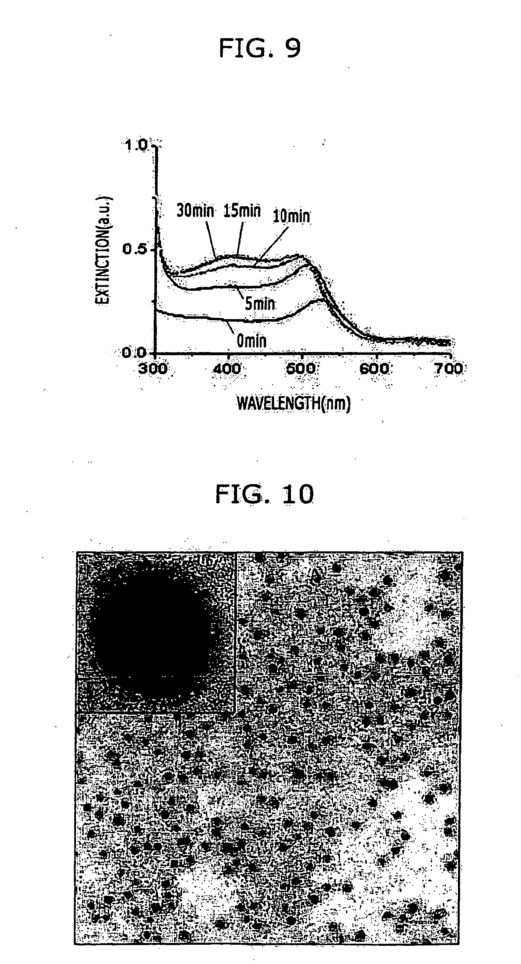

Fig. 9 shows variation in extinction of the solution measured by the UV-visible spectrometer with respect to time. As can be seen from Fig. 9, the extinction was not substantially varied after reaction for about 30 minutes.

Furthermore, theshape and size of the prepared Au/Ag core-shell composite were confirmed using a TEM, and the result was shown in Fig. 10. An image shown on the left upper side of Fig. 10 is an enlarged image of the composite.

As a result of the TEM analysis, the Au/Ag core-shell composite of Example 4 was spherical in shape and was about 20 nm to about 22 nm in size, and the Ag nanoparticle layer was about 5 nm to about 7 nm in thickness.

**Example 5: Bonding of Protein A (Spacer Molecule) and Antibody (Receptor) on Surface of Au Nanoparticle**

The solution where about 10 *µ*g of protein A was dissolved was added to 1 mℓ of the 3.8 nM solution of Au nanoparticle with a diameter of 15 nm and was mixed by shaking in a phosphate buffer solution (pH 4-10) at room temperature for 1 hour. About 10 pug of antibody (protein receptor) purchased from piercenet.com was added to the solution and was mixed by shaking at room temperature for 1-5 hours. After adding a surfactant such as BSA or SDS and additionally leaving the solution for more than 12 hours, the solution was centrifuged to remove the supernatant. In this way, the antibody thatwas not bonded with the protein A bonded on the surface of the Au nanoparticle was removed. The procedure of diluting the solution by adding 1 mℓ of 0.15 M phosphate (10 mM PB, 0.15M NaCl) was performed two times.

In this way, the protein A was bonded on the surface of the Au nanoparticle, and the antibody was bonded with the protein A.

**Examples 6: Preparation of Au/Ag Core-shell Composite bonded with Protein A and Antibody**

The concentration of the Au nanoparticle solution where the protein A was bonded on its surface and the antibody was bonded with the protein A, which was synthesized in Example 5, was calculated using the extinction measured by the UV-visible spectrometer. The concentration of the solution was adjusted to 1 nM by concentrating or diluting the solution according to the result.

To 250 *µ*ℓ. of the solution, 12 *µ*ℓ of AgNO₃ solution diluted 10 times with distilled water, and 12 *µ*ℓ of hydroquinone solution diluted 10 times with distilled water were sequentially added and then agitated for 30 minutes.

Thereafter, the extinction of the solution was measured by the UV-visible spectrometer. After leaving the solution at room temperature till there is no change in the extinction, it was centrifuged to remove the supernatant and was diluted with distilled water. Then, the solution was again centrifuged to remove the supernatant and was diluted with 250 *µ*ℓ of distilled water.

In this way, the Au/Ag core-shell composite bonded with the antibody in accordance with the embodiment of the present invention was prepared.

Fig. 2 is a schematic view showing the Au/Ag core-shell composite, and Fig. 4 is a schematic view showing the method for preparing the Au/Ag core-shell composite.

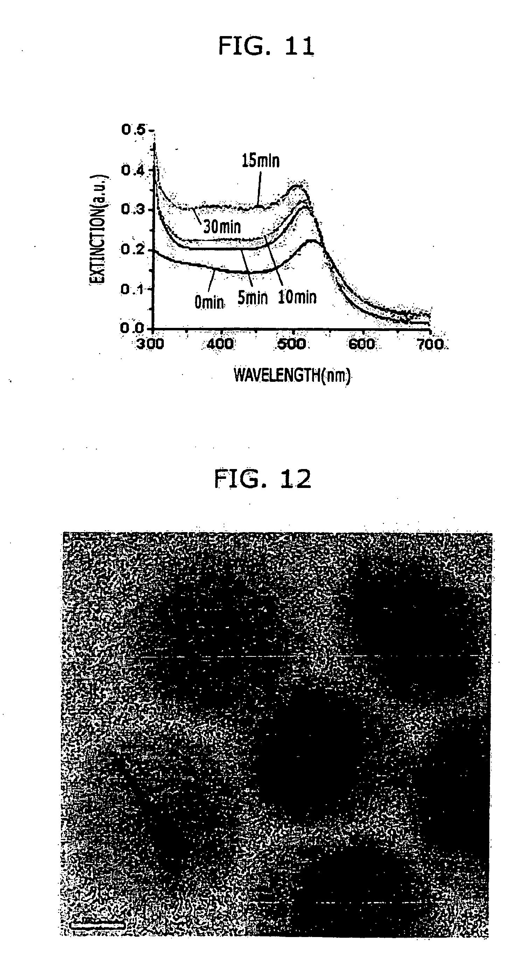

Fig. 11 shows variation in extinction of the solution measured by the UV-visible spectrometer with respect to time. As can be seen from Fig. 11, the extinction was not substantially varied after reaction for about 30 minutes.

Furthermore, theshape and size of the prepared Au/Ag core-shell composite were confirmed using a TEM and the result was shown in Fig. 12. As a result of the TEM analysis, the prepared Au/Ag core-shell composite was spherical in shape and was about 16 nm to about 17 nm in size, and the Ag nanoparticle layer was about 1.5 nm.

Furthermore, according to the EDX analysis, silver (Ag) atoms and gold (Au) atoms in the Au/Ag core-shell composite were 25 % and 75 %, respectively. This result was identical to thatof the Au/Ag core-shell composite bonded with oligonucleotide of example 3 (see Fig. 8).

**Example 7: Comparison of Au/Ag Core-shell Composite of the above examples and Mixture of pure Au nanoparticle and Au nanoparticle**

To confirm the structure of the Au/Ag core-shell composite of Example 3, the UV extinction of the Au/Ag core-shell composite of Example 3 was compared with the UV extinction of the simple mixture of pure Au nanoparticle with a size of 15 nm and pure Ag nanoparticle with a size of 15 nm.

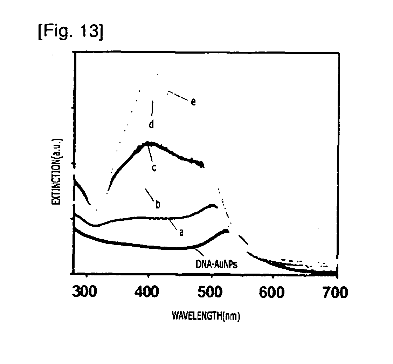

The UV extinction of the Au/Ag core-shell composite, which was prepared according to Example 3 except that the thickness of the Ag nanoparticle layer was changed by adjusting amounts of AgNO₃ and hydroquinone as shown in Table 1 below, was measured and shown in Fig. 13. Table 1 shows variation in UV extinction of Au/Ag core-shell composite according to amounts of AgNO₃ and hydroquinone.

Table 1

**[Table 1]**

| Amount of AgNO₃ (µl) | Amount of hydroquinone (µl) | Extinction data |
|---|---|---|
| 1.2 | 1.2 | Fig. 13-a |
| 2.0 | 2.0 | Fig. 13-b |
| 2.4 | 2.4 | Fig. 13-c |
| 3.2 | 3.2 | Fig. 13-d |
| 4.0 | 4.0 | Fig. 13-e |

In the case of the Au/Ag core-shell composite in accordance with the present invention, as shown in Fig. 13, a blue shift occurred at 520 nm, which is the maximum absorption peak of the Au nanoparticle, according to the thickness of the Ag nanoparticle layer, and the maximum absorption peak moved to 500 nm, 490 nm, and so on. The characteristic maximum absorption peak of the Ag nanoparticle occurred at 400 nm. Also, it was observed that the intensity of the extinction was changed according to the thickness of the Ag nanoparticle layer.

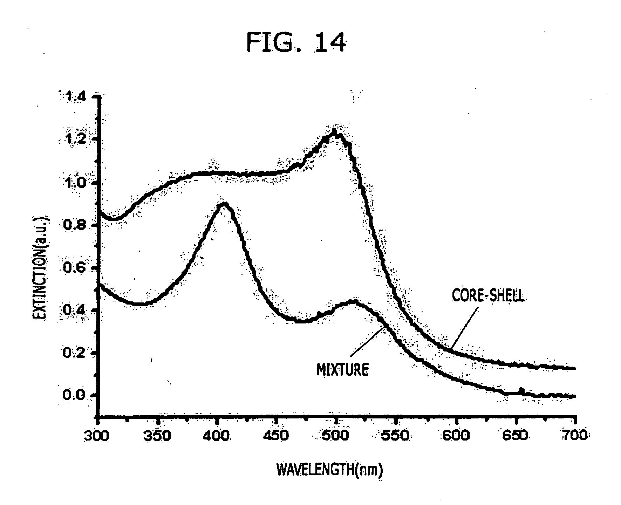

Meanwhile, Fig. 14 shows the comparison of UV extinction of the Au/Ag core-shell composite indicated by "a" of Fig. 13 and the simple mixture of the pure Au nanoparticle with a size of 15 nm and the pure Ag nanoparticle with a size of 15 nm.

As can be seen from Fig. 14, unlike the Au/Ag core-shell composite in accordance with the present invention, the maximum absorption peaks of the simple mixture occurred at the characteristicmaximum absorption peaks of the Au nanoparticle and the Ag nanoparticle, that is, 400 nm corresponding to the Ag nanoparticle with a size of 15 nm and 520 nm corresponding to the Au nanoparticle with a sizeof 15 nm. In the Au/Ag core-shell composite, however, the blue shift occurred from about 520 nm to about 510 nm in thecase of the maximum absorption peak of the Au nanoparticle, and the wide peak occurred at about 400 nm in the case of the Ag nanoparticle. Therefore, it was confirmed that the core-shell composite in accordance with the present invention does not exist in a form of the simple mixtureof the Au nanoparticle and the Ag nanoparticle, but exists in a form of one core-shell nanoparticle.

**Example 8: Stability Test**

A stability test was performed with respect to temperature and time in such a state that the Au/Ag core-shell composite bonded with oligonucleotide, which was prepared in Example 3, was kept in 0.3 M phosphate buffer solution.

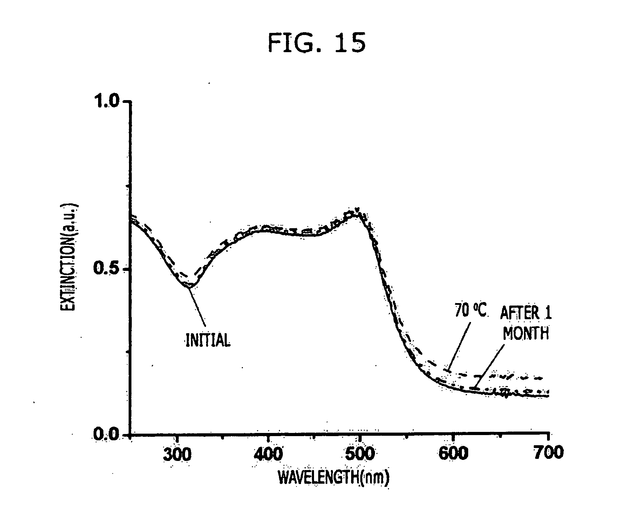

The result of the stability test is shown in Fig. 15.

As shown in Fig. 15, there was no difference in the UV extinction measured before and after the temperature of the solution increased to 70°C. Also, there was no difference in the UV extinction measured before and after leaving it at room temperature for 1 month. The same test result was obtained in the Au/Ag core-shell composite boned with protein A and antibody, which was prepared in Example 6.

As can be inferred from the result of the stability test, the spacer site or the spacer molecule of the receptor in the Au/Ag core-shell composite of the present invention was bonded on the surface of the Au nanoparticle and embedded into the Ag nanoparticle layer, and the target material recognition site of the receptor was exposed to the outside of the Ag nanoparticle layer, and thus, superior stability with respect to temperature and time were exhibited.

**Example 9: Colorimetric Assay test**

As described in Example 3, the Au/Ag core-shell composite where oligonucleotide A and oligonucleotide B were bonded was prepared.

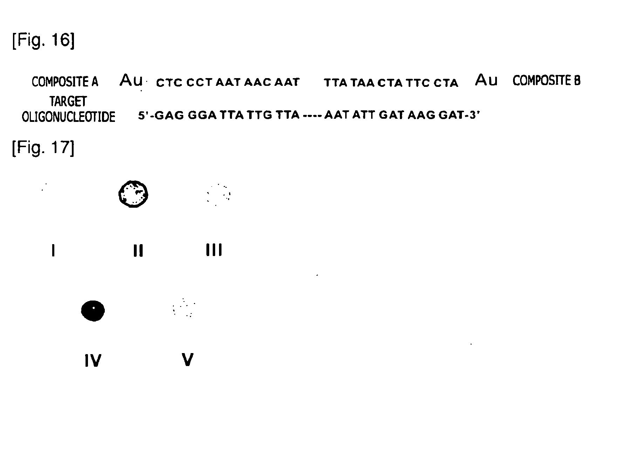

Fig. 16 shows the base sequence of the oligonucleotides A and B contained in the Au/Ag core-shell composite, and the target oligonucleotide having a complementary base sequence. 300 *µ*ℓ of Au/Ag core-shell composite A (1.5 pmol) bonded with oligonucleotide A dissolved in 0.3 M phosphate buffer solution was mixed with 375 *µ*ℓ of Au/Ag core-shell composite B (1.5 pmol) bonded with oligonucleotide B dissolved in phosphate buffer solution. 6.0 *µ*ℓ (10 µM) of target oligonucleotide was added to the mixed solution, the temperature of the mixed solution increased to 70°C, and then gradually decreased to room temperature. After abouttwo hours, the solution changed from the initial orange color to the dark purple color.

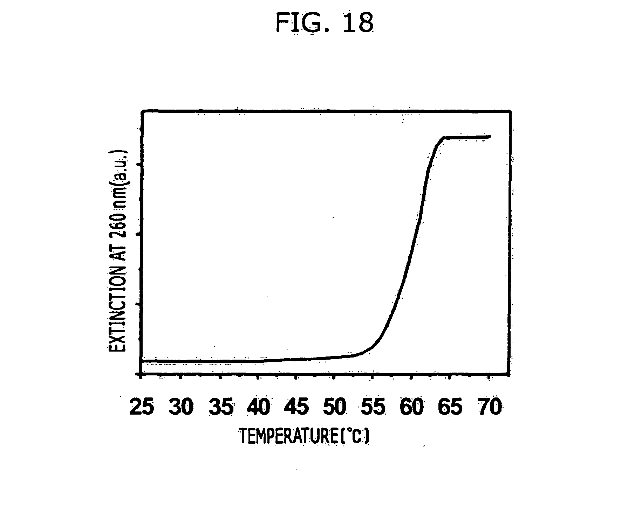

The change of the color could be observed more clearly by dropping 2 *µ*ℓ of the solution on a C I 8-coated glass plate. An image of Fig. 17-I shows the color (green) of the 15 nm Ag nanoparticle; an image of Fig. 17-II shows the color (purple) of the 15 nm Au nanoparticle; an image of Fig. 17-III shows the color (orange) of the 15 nm Au/Ag core-shell composites in accordance with the present invention; an image of Fig. 17-IV shows the color of the state where the Au/Ag core-shell composites were complementarily bonded with the target oligonucleotide base sequence and aggregated and an image of Fig. 17-V shows the color of the state where the temperature of the aggregated Au/Ag core-shell composite solution increased above the melting point (in this case, 53°C) of the oligonucleotide base sequence, so that the complementary hydrogen bond was broken to make the distance of the aggregated Au/Ag core-shell composites apart from each other, and thus, the color was restored to the original color. Fig. 18 shows the above experimental results as the variation of the melting point of the aggregated Au/Ag core-shell composite with respect to time. Specifically, Fig. 9C shows the extinction measured at 260 nm of the aggregated Au/Ag core-shell composite while increasing the temperature from room temperature to 70°C. It can be seen from Fig. 18 that the bonded oligonucleotide was separated in a range of about 55°C to about 65°C.

According to the result of the colorimetric assay test, the target material recognition site of the receptor was not embedded into the Ag nanoparticle layer, but was exposed to the outside of the Au nanoparticle layer. Thus, the normal target recognition function was carried out.

**Example 10: Preparation of Au/Ag Core-shell Composite when Au nanoparticle is a combination of two or more particles**

The oligonucleotides A and B of Example 1 were bonded on the Au nanoparticle according to Example 2. 6.0 *µ*ℓ of 10 µM target oligonucleotide (see Fig. 16) was added to the mixed solution containing Au nanoparticle bonded with oligonucleotide A and Au nanoparticle bonded with oligonucleotide B, which were dissolved in 0.3 M phosphate buffer solution. The temperature of the mixed solution increased to 70°C and then gradually decreaseddown to room temperature. It was observed through the TEM that the separate Au nanoparticles formed a dimer after about 2 hours.

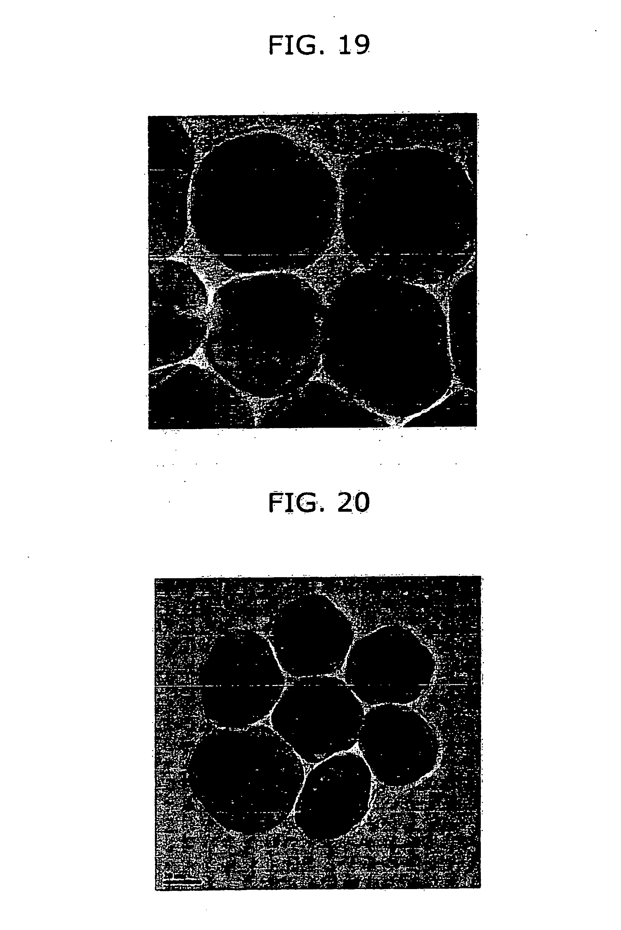

To the 250 *µ*ℓ of the solution, 50 *µ*ℓ of AgNO₃ (10⁻³ M) and 50 *µ*ℓ of hydroquinone solution were added and then agitated for 3 hours. As a result of observing the progress of the reaction through the UV-visible spectroscopy, the extinction was increased at 400 nm as shown in Fig. 13. Moreover, as an observation result using the TEM, the Ag nanoparticle layer was formed even in the dimer and the combination of the dimer or more (see Figs. 19 and 20).

In accordance with the embodiments of the present invention, even in the Au nanoparticle having the combination of the dimer or more, the Ag nanoparticle layer forming the shell can be formed while effectively adjusting its thickness. Although the method for preparing the dimer has been described as the method using oligonucleotide, it is merely exemplary and the present invention is not limited thereto.

The present application contains subject matter related to Korean Patent Application No. 10-2008-0042374 and 10-2009-0039472, filed in the Korean Intellectual Property Office on May 7, 2008, and May 6, 2009, respectively, the entire contents of which is incorporated herein by reference.

## Claims

1. An Au/Ag core-shell composite-receptor conjugate, comprising:
an Au nanoparticle
an Ag nanoparticle layer surrounding the Au nanoparticle; and
a receptor having a target material recognition site bondable or reactable with a target material,
wherein one end of the receptor is bonded on the surface of the Au nanoparticle, so that a portion of the receptor is embedded into the Ag nanoparticle layer, and the target material recognition site is exposed to the outside of the Ag nanoparticle layer.

2. The Au/Ag core-shell composite-receptor conjugate of claim 1, wherein the receptor further comprises a spacer element, one end of the spacer element being bonded with the surface of the Au nanoparticle, another end of the spacer element being bonded on the target material recognition site, wherein the spacer site comprises:
a base sequence consisting of one base selected from adenine, guanine, cytosine, and thymine;
polyethylene glycol (PEG); or
a combination of the base sequence and the polyethylene glycol (PEG).

3. The Au/Ag core-shell composite-receptor conjugate of claim 1, further comprising a spacer molecule that mediates the bond between the receptor and the Au nanoparticle, wherein the spacer molecule comprises one or more selected from the group consisting of protein A, protein G, and protein A/G.

4. The Au/Ag core-shell composite-receptor conjugate of any one of claims 1 to 3, wherein the receptor comprises one or more selected from the group consisting of enzyme substrate, ligand, amino acid, peptide, protein, antibody, nucleic acid, oligonucleotide, lipid, cofactor, and carbohydrate.

5. The Au/Ag core-shell composite-receptor conjugate of any one of claims 1 to 3, wherein the receptor, the spacer element of the receptor, or the spacer molecule further comprises a functional group that mediates the bond with the Au nanoparticle, wherein the functional group is selected from one or more of the group consisting of an amine group, a carboxyl group, a thiol group and a phosphate group.

6. The Au/Ag core-shell composite-receptor conjugate of any one of claims 1 to 3, wherein the Au nanoparticle comprises one nanoparticle or a combination of two or more nanoparticles.

7. A method for preparing an Au/Ag core-shell composite-receptor conjugate, the method comprising:
bonding one end of a receptor, which has a target material recognition site bondable or reactable with a target material, on the surface of an Au nanoparticle;
forming an Ag nanoparticle layer on the surface of the Au nanoparticle so that a portion of the receptor is embedded into the Ag nanoparticle layer, and the target material recognition site of the receptor is exposed to the outside of the Ag nanoparticle layer.

8. The method of claim 7, further comprising connecting a spacer element to the target material recognition site of the receptor before said bonding one end of the receptor on the surface of the Au nanoparticle,
wherein said bonding one end of the receptor is performed by bonding one end of the spacer element of the receptor on the surface of the Au nanoparticle, and
said forming the Ag nanoparticle layer comprises forming an Ag nanoparticle layer on the surface of the Au nanoparticle so that the spacer element of the receptor is embedded into the Ag nanoparticle layer, and the target material recognition site of the receptor is exposed to the outside of the Ag nanoparticle layer.

9. The method of claim 7, further comprising attaching a spacer molecule to the surface of the Au nanoparticle before said bonding one end of the receptor on the surface of the Au nanoparticle,
wherein said bonding one end of the receptor is performed by bonding the spacer molecule, which is attached to the surface of the Au nanoparticle, with the receptor, and
said forming the Ag nanoparticle layer comprises forming the Ag nanoparticle layer on the surface of the Au nanoparticle so that the spacer molecule is embedded into the Ag nanoparticle layer, and the target material recognition site of the receptor is exposed to the outside of the Ag nanoparticle layer.

10. The method of any one of claims 7 to 9, wherein the bond of the receptor, the spacer element of the receptor, or the spacer molecule on the surface of the Au nanoparticle is performed by mediation of a functional group, wherein the functional group is selected from one or more of the group consisting of an amine group, a carboxyl group, a thiol group and a phosphate group.

11. A biosensor comprising the Au/Ag core-shell composite-receptor conjugate of any one of claims 1 to 3, wherein the biosensor is for detecting a target material bondable or reactable with the target material recognition site of the receptor of the Au/Ag core-shell composite-receptor conjugate.

## Patentansprüche

1. Au/Ag-Kern-Hülle-Verbundstoff-Rezeptorkonjugat, umfassend:
ein Au-Nanopartikel
eine Ag-Nanopartikelschicht, die das Au-Nanopartikel umgibt; und
einen Rezeptor mit einer mit einem Zielmaterial bindungs- oder reaktionsfähigen Zielmaterial-Erkennungsstelle,
wobei ein Ende des Rezeptors auf der Oberfläche des Au-Nanopartikels gebunden ist, sodass ein Anteil des Rezeptors in die Ag-Nanopartikelschicht eingebettet ist, und die Zielmaterial-Erkennungsstelle der Außenseite der Ag-Nanopartikelschicht ausgesetzt ist.

2. Au/Ag-Kern-Hülle-Verbundstoff-Rezeptorkonjugat nach Anspruch 1, wobei der Rezeptor ferner ein Spacer-Element umfasst, wobei ein Ende des Spacer-Elements mit der Oberfläche des Au-Nanopartikels verbunden ist, wobei ein anderes Ende des Spacer-Elements auf der Zielmaterial-Erkennungsstelle gebunden ist, wobei die Spacer-Region Folgendes umfasst:
eine Basensequenz, bestehend aus einer Base, die aus Adenin, Guanin, Cytosin und Thymin ausgewählt ist;
Polyethylenglykol (PEG); oder
eine Kombination aus der Basensequenz und dem Polyethylenglykol (PEG).

3. Au/Ag-Kern-Hülle-Verbundstoff-Rezeptorkonjugat nach Anspruch 1, ferner umfassend ein Spacer-Molekül, das die Bindung zwischen dem Rezeptor und dem Au-Nanopartikel vermittelt, wobei das Spacer-Molekül eines oder mehr umfasst, das/die aus der Gruppe ausgewählt ist/sind, bestehend aus Protein A, Protein G und Protein A/G.

4. Au/Ag-Kern-Hülle-Verbundstoff-Rezeptorkonjugat nach einem der Ansprüche 1 bis 3, wobei der Rezeptor einen oder mehr umfasst, der/die aus der Gruppe ausgewählt ist/sind, bestehend aus einem Enzymsubstrat, einem Liganden, einer Aminsäure, einem Peptid, einem Protein, einem Antikörper, einer Nukleinsäure, einem Oligonukleotid, einem Lipid, einem Cofaktor und einem Kohlenhydrat.

5. Au/Ag-Kern-Hülle-Verbundstoff-Rezeptorkonjugat nach einem der Ansprüche 1 bis 3, wobei der Rezeptor, das Spacer-Element des Rezeptors oder das Spacer-Molekül ferner eine funktionelle Gruppe umfasst, welche die Bindung mit dem Au-Nanopartikel vermittelt, wobei die funktionelle Gruppe aus einer oder mehr der Gruppen ausgewählt ist, bestehend aus einer Amingruppe, einer Carboxylgruppe, einer Thiolgruppe und einer Phosphatgruppe.

6. Au/Ag-Kern-Hülle-Verbundstoff-Rezeptorkonjugat nach einem der Ansprüche 1 bis 3, wobei das Au-Nanopartikel ein Nanopartikel oder eine Kombination aus zwei oder mehr Nanopartikeln umfasst.

7. Verfahren zur Herstellung eines Au/Ag-Kern-Hülle-Verbundstoff-Rezeptorkonjugats, wobei das Verfahren Folgendes umfasst:
Binden eines Endes eines Rezeptors, der eine mit einem Zielmaterial bindungs- oder reaktionsfähige Zielmaterial-Erkennungsstelle aufweist, an die Oberfläche eines Au-Nanopartikels;
Bilden einer Ag-Nanopartikelschicht auf der Oberfläche des Au-Nanopartikels, sodass ein Anteil des Rezeptors in die Ag-Nanopartikelschicht eingebettet ist und die Zielmaterial-Erkennungsstelle des Rezeptors der Außenseite der Ag-Nanopartikelschicht ausgesetzt ist.

8. Verfahren nach Anspruch 7, ferner umfassend die Verbindung eines Spacer-Elements mit der Zielmaterial-Erkennungsstelle des Rezeptors vor der Bindung eines Endes des Rezeptors an die Oberfläche des Au-Nanopartikels,
wobei die Bindung eines Endes des Rezeptors durch Bindung eines Endes des Spacer-Elements des Rezeptors an die Oberfläche des Au-Nanopartikels durchgerührt wird, und
das Bilden der Ag-Nanopartikelschicht das Bilden einer Ag-Nanopartikelschicht auf der Oberfläche des Au-Nanopartikels umfasst, sodass das Spacer-Element des Rezeptors in die Ag-Nanopartikelschicht eingebettet ist, und die Zielmaterial-Erkennungsstelle des Rezeptors der Außenseite der Ag-Nanopartikelschicht ausgesetzt ist.

9. Verfahren nach Anspruch 7, ferner umfassend das Anlagern eines Spacer-Moleküls an die Oberfläche des Au-Nanopartikels vor der Bindung eines Endes des Rezeptors an die Oberfläche des Au-Nanopartikels,
wobei die Bindung eines Endes des Rezeptors durch Bindung des an die Oberfläche des Au-Nanopartikels angelagerten Spacer-Moleküls mit dem Rezeptor durchgeführt wird und
das Bilden der Ag-Nanopartikelschicht das Bilden der Ag-Nanopartikelschicht auf der Oberfläche des Au-Nanopartikels umfasst, sodass das Spacer-Molekül in die Ag-Nanopartikelschicht eingebettet ist und die Zielmaterial-Erkennungsstelle des Rezeptors der Außenseite der Ag-Nanopartikelschicht ausgesetzt ist.

10. Verfahren nach einem der Ansprüche 7 bis 9, wobei die Bindung des Rezeptors, des Spacer-Elements des Rezeptors oder des Spacer-Moleküls an die Oberfläche des Au-Nanopartikels durch Vermittlung einer funktionellen Gruppe durchgeführt wird, wobei die funktionelle Gruppe aus einer oder mehr der Gruppen ausgewählt ist, bestehend aus einer Amingruppe, einer Carboxylgruppe, einer Thiolgruppe und einer Phosphatgruppe.

11. Biosensor umfassend das Au/Ag-Kern-Hülle-Verbundstoff-Rezeptorkonjugat nach einem der Ansprüche 1 bis 3, wobei der Biosensor zum Nachweis eines mit der Zielmaterial-Erkennungsstelle des Rezeptors des Au/Ag-Kern-Hülle-Verbundstoff-Rezeptorkonjugats bindungs- oder reaktionsfähigen Zielmaterial dient.

## Revendications

1. Conjugué d'un composite coeur/enveloppe Au/Ag-récepteur, comprenant:
une nanoparticule Au
une couche de nanoparticules Ag entourant la nanoparticule Au; et
un récepteur ayant un site de reconnaissance de substance cible pouvant se lier à/ou réagir avec une substance cible,
où une extrémité du récepteur est liée sur la surface de la nanoparticule Au, de sorte qu'une partie du récepteur est noyée dans la couche de nanoparticules Ag et que le site de reconnaissance de substance cible est exposé à l'extérieur de la couche de nanoparticules Ag.

2. Conjugué d'un composite coeur/enveloppe Au/Ag-récepteur selon la revendication 1, dans lequel le récepteur comprend en outre un élément espaceur, une extrémité de l'élément espaceur étant liée sur la surface de la nanoparticule Au, une autre extrémité de l'élément espaceur étant liée au site de reconnaissance de substance cible, où le site de l'espaceur comprend:
une séquence de base consistant en une base sélectionnée parmi adénine, guanine, cytosine et thymine;
du polyéthylène glycol (PEG); ou bien
une combinaison de la séquence de base et de polyéthylène glycol (PEG).

3. Conjugué d'un composite coeur/enveloppe Au/Ag-récepteur selon la revendication 1, comprenant en outre une molécule espaceur par la médiation de laquelle la liaison entre le récepteur et la nanoparticule Au se fait, où la molécule espaceur comprend une ou plusieurs protéines sélectionnées parmi le groupe consistant en protéine A, protéine G et protéine A/G.

4. Conjugué d'un composite coeur/enveloppe Au/Ag-récepteur selon l'une quelconque des revendications 1 à 3, dans lequel le récepteur comprend un ou plusieurs éléments sélectionnés parmi le groupe consistant en substrat enzymatique, ligand, acide aminé, peptide, protéine, anticorps, acide nucléique, oligonucléotide, lipide, cofacteur et hydrate de carbone.

5. Conjugué d'un composite coeur/enveloppe Au/Ag-récepteur selon l'une quelconque des revendications 1 à 3, dans lequel le récepteur, l'élément espaceur du récepteur ou la molécule espaceur comprend en outre un groupe fonctionnel par la médiation duquel la liaison avec la nanoparticule Au se fait, où le groupe fonctionnel est sélectionné parmi un ou plusieurs éléments du groupe consistant en un groupe amine, un groupe carboxyle, un groupe thiol et un groupe phosphate.

6. Conjugué d'un composite coeur/enveloppe Au/Ag-récepteur selon l'une quelconque des revendications 1 à 3, dans lequel la nanoparticule Au comprend une nanoparticule ou bien une combinaison de deux nanoparticules ou plus.

7. Procédé de préparation d'un conjugué d'un composite coeur/enveloppe Au/Ag-récepteur, le procédé comprenant:
lier une extrémité d'un récepteur qui a un site de reconnaissance de substance cible pouvant être lié à/ ou réagir avec une substance cible, sur la surface d'une nanoparticule Au;
former une couche de nanoparticules Ag sur la surface de la nanoparticule Au de sorte qu'une partie du récepteur est noyée dans la couche de nanoparticules Ag et le site de reconnaissance de substance cible du récepteur est exposé à l'extérieur de la couche de nanoparticules Ag.

8. Procédé selon la revendication 7, comprenant en outre la connexion d'un élément espaceur au site de reconnaissance de substance cible du récepteur avant ladite liaison d'une extrémité du récepteur sur la surface de la nanoparticule Au,
dans lequel ladite liaison d'une extrémité du récepteur est effectuée en liant une extrémité de l'élément espaceur du récepteur sur la surface de la nanoparticule, et
ladite formation d'une couche de nanoparticules Ag comprend la formation d'une couche de nanoparticules Ag sur la surface de la nanoparticule Au de sorte que l'élément espaceur du récepteur est noyé dans la couche de nanoparticules Ag et que le site de reconnaissance de substance cible du récepteur est exposé à l'extérieur de la couche de nanoparticules Ag.

9. Procédé selon la revendication 7, comprenant en outre l'attachement d'une molécule espaceur à la surface de la nanoparticule Au avant de lier une extrémité du récepteur sur la surface de la nanoparticule Au,
où ladite liaison d'une extrémité du récepteur est effectuée en liant la molécule espaceur, qui est attachée à la surface de la nanoparticule Au, au récepteur, et
ladite formation d'une couche de nanoparticules Ag comprend la formation de la couche de nanoparticules Ag à la surface de la nanoparticule Au de sorte que la molécule espaceur est noyée dans la couche de nanoparticules Ag et que le site de reconnaissance de substance cible est exposé à l'extérieur de la couche de nanoparticules Ag.

10. Procédé selon l'une quelconque des revendications 7 à 9, dans lequel la liaison du récepteur, de l'élément espaceur ou de la molécule espaceur du récepteur sur la surface de la nanoparticule Au est effectuée par la médiation d'un groupe fonctionnel, où le groupe fonctionnel est sélectionné parmi un ou plusieurs du groupe consistant en un groupe amine, un groupe carboxyle, un groupe thiol et un groupe phosphate.

11. Biocapteur comprenant le conjugué d'un composite coeur/enveloppe Au/Ag-récepteur selon l'une quelconque des revendications 1 à 3, dans lequel le biocapteur sert à détecter une substance cible liable ou pouvant réagir avec le site de reconnaissance de substance cible du récepteur du conjugué d'un composite coeur/enveloppe Au/Ag-récepteur.
